# EUROPEAN PATENT SPECIFICATION

(11) **EP 3 952 827 B1**
(45) Date of publication and mention of the grant of the patent: **07.05.2025**
(21) Application number: 20786949.6
(22) Date of filing: 09.04.2020
(51) Int. Cl.: A61K 8/24, A61K 8/27, A61K 8/34, A61K 8/20, A61K 8/73, A61Q 11/00

(54) **ORAL RINSE COMPOSITIONS FOR REDUCING PATHOGENS UNDERLYING AND FOR TREATING PERIODONTAL DISEASE AND ORAL MALODOR IN THE CANINE**
MUNDSPÜLZUSAMMENSETZUNGEN ZUR VERMINDERUNG DER ZUGRUNDE LIEGENDEN PATHOGENE UND ZUR BEHANDLUNG VON PARODONTOSE UND MUNDGERUCH BEI HUNDEN
COMPOSITIONS DE RINÇAGE BUCCAL POUR RÉDUIRE DES PATHOGÈNES SOUS-JACENTS ET POUR TRAITER LA MALADIE PARODONTALE ET LA MAUVAISE HALEINE CHEZ LE CHIEN

(30) Priority: 10.04.2019 US 201962832100 P
(43) Date of publication of application: 16.02.2022
(73) Proprietor: Black Tulip Management, Inc., New York, NY 10016 (US)
(72) Inventor: SCALA, Anthony, Jamesville, NY 13078 (US); MALPESO, Pasquale, New York, NY 10011 (US); NICKELS, Michael, L., Nashville, TN 37205 (US); CROSSETTI, Henry, W., Kiawah Island, SC 29455 (US); WILCOX-ADELMAN, Sarah, Demarest, NJ 07627 (US)
(74) Representative: HGF
(86) International application number: PCT/US2020/027506
(87) International publication number: WO 2020/210518

(56) References cited:
- EP-A1- 2 392 343
- WO-A1-2019/046841
- WO-A2-02/02061
- GB-A- 2 289 841
- US-A1- 2001 006 624
- US-A1- 2002 182 267
- US-A1- 2006 193 790
- US-A1- 2014 341 819

## Description

### BACKGROUND OF THE INVENTION

Periodontal disease is one of the most common infections found in canines. It has been estimated that 80% of all canines suffer from periodontal disease after age three.

Periodontal disease is the result of the accumulation of bacteria containing plaque on the teeth and the surrounding soft tissue. Bacterial accumulation in the canine is normal, and is removed as the result of several factors including salivary flow, abrasive food products and contact by the tongue and checks against the surfaces of the teeth. However, the abrasive action of the tongue, which assists in limiting the bacterial accumulation, is effective mainly on the lingual surfaces of the teeth, and often does not extend to all areas (e.g., the front surfaces of the teeth) within the oral cavity due to limitations on tongue movement.

The progression of periodontal disease in the canine starts with a pellicle formation, which arises from salivary proteins that attach to the surface of the teeth. Once formed, oral bacteria start to attach to this pellicle, forming a biofilm. As the bacterial population increases in this biofilm, plaque is formed. Plaque, if left undisturbed for 24 to 48 hours, will cause inflammation to the soft tissue surrounding the teeth, known as gingivitis. This is considered the first stage of periodontal disease, but may be reversed with proper care. Left untreated, plaque will eventually become hard due to calcification from minerals found in saliva, this being known as tartar or calculus. Unlike plaque or biofilms, tartar cannot be removed by abrasion (brushing), but requires professional scaling for removal. Plaque and tartar, if undisturbed, accumulate under the gum tissue, and cause permanent destruction of the hard and soft tissue supporting the teeth, this permanent destruction referred to as periodontitis.

Moreover, the gram-negative anaerobic bacteria which accumulates under the soft tissue supporting the teeth also function to break down any sloughed cells and their sulfur containing amino acids found in oral organic matter, such as cellular debris, food particles and salivary proteins. This serves to release sulfide gases into the oral cavity, which constitute the noxious odors in the canine breath. These odors intensify and become more noxious as the disease progresses and become more severe. The canine is not aware of this, however, due to canine olfactory adaptation, but this odor is readily apparent to humans.

One dilemma facing veterinarians is that the bacteria which underlie periodontal disease are also present in the normal flora in the canine. Thus, attempting to control these bacteria via the use of antibiotics would be detrimental to the native canine flora.

The current standard of care for oral health in the canine is daily brushing and, in advanced disease states, professional cleaning under general anesthesia. However, daily brushing can be tedious and difficult in the canine for both the canine and the owner because this is an atypical procedure. Professional cleaning, although effective, is costly and, due to the need for general anesthesia, can be dangerous for the canine. In addition, such cleaning does not provide a long-term solution, as supragingival plaque has been found in canines in as little as 24 hours following a professional cleaning.

### BRIEF SUMMARY OF THE INVENTION

The invention relates to formulations for retarding the advance of, or treating, periodontal disease in a canine, methods for the preparation of these formulations, as well as related methods for using of these formulations.

The present invention is that which is laid out in the appended claims.

### DETAILED DESCRIPTION OF THE INVENTION

The present invention provides formulations, and related methods, suitable for application onto the canine oral mucosa and teeth wherein, after application of one of the formulations thereon, a reduction in the concentration of bacteria on teeth and/or on the surrounding soft tissue is provided. This reduction in bacteria is believed to retard the advance of, or treat existing, periodontal disease.

The formulation of the invention is an aqueous solution or dispersion comprising, consisting essentially of, or consisting of, hypobromite salt, a chlorite salt, water, and a pH adjusting agent, as well as a viscosity enhancer, wherein the formulation has a pH of from about 4 to about 9, and desirably is non-acidic, having a pH of from about 7 to about 9.

The formulation is prepared via mixing a plurality of components to provide an aqueous solution or dispersion, the components comprising, a hypobromite salt, a chlorite salt, water, and a pH adjusting agent, and a viscosity enhancer wherein the resulting formulation has a pH of from about 4 to about 9,

As the inventive formulation is intended for application onto the oral mucosa, each ingredient used to prepare the formulation, and the resulting formulation itself, should be non-toxic (or include ingredients at concentrations that are deemed to be non-toxic) by relevant regulatory authorities.

The formulations of the present invention were found to provide various advantages relative to existing formulations. By way of example, it was discovered that certain embodiments of the formulations are effective in reducing bacterial count upon contact with the oral mucosa (e.g., desirably possessing an oxidation reduction potential (ORP) of at least about 650 mV) despite using relatively low concentrations of chlorite salt and hypobromite salt. Further, and desirably, certain embodiments of the formulations provide this antibacterial activity at a non-acidic pH, e.g., from 7 to about 10. Providing this degree of antibacterial activity at a non-acidic pH is highly desirable when the formulations are used to contact oral mucosa, and particularly when they are used to treat periodontal disease as acidic liquids exacerbate this condition and associated discomfort.

It was further discovered that the formulations retain their activity (e.g., ORP) for an extended period of time, e.g., for up to about 7, 14, 30, 45, 60, 90, 120, 150 or 180 days, even in formulations that are prepared and packaged at a non-acidic pH. Desirably, and to enhance activity retention, the formulations may be packaged within a UV-resistant container, e.g., a tinted or opaque container, re-sealable if desired. In addition, it is believed that the hypobromite salt is stabilized in the formulations, even at non-acidic pHs, wherein there exists relatively minimal degradation of the hypobromite salt into a bromide and/or bromate salt after formulation preparation. This stability is desirably also retained at temperatures ranging from about 4°C to about 25°C with refrigeration assisting in enhancing stability. When packaged, it is further desirable for any headspace in the container to be filled with an inert gas (e.g., nitrogen) during packaging, and more preferably under (limited) pressure, which pressure, it is believed, will assist in retaining the chlorine dioxide in solution prior to initial use. Packaging of the formulation for single use (unit dose packaging) is also contemplated.

Each of the inventive aqueous formulations is prepared with a hypobromite salt. This salt may include one of a variety of cations, e.g., alkali and alkali earth metals salts such as lithium, sodium, potassium, calcium, magnesium and zinc, is desirably a sodium and/or potassium salt, and is more desirably the sodium salt. In preparing the formulations, the hypobromite salt may be present in the aqueous dispersion or solution in weight amounts ranging from about 1, about 50, about 100, about 250, about 500, about 750, about 1,000, about 1,250, about 1,500, about 1,750 or about 2,000 ppm to about 3,000, about 5,000, about 10,000, about 15,000, about 20,000, about 25,000, about 30,000, about 40,000, about 50,000, about 60,000, about 70,000, about 80,000, about 90,000 or about 100,000 ppm, based on the total weight of the formulation, and desirably ranges from about 1, 10, 25, 50, 75, 100 or 125 to about 150, 250, 500, 750, 1,000, 1,200 or 1,500 based, again, on the total weight of the formulation. While not desiring to be bound to a particular theory, it is believed that inclusion of the hypobromite salt is believed to provide the formulation with a pathogenic effect on bacteria, viruses and fungi, e.g., via oxidation of the cell membrane therein. It is further believed that this oxidation causes instability and ultimately disintegration of that membrane. Also, because the formulation does not require uptake and metabolism, bacteria, viruses and fungi cannot develop a resistance to the formulation.

The formulations are desirably prepared using sodium hypobromite as the sole hypobromite salt, and more preferably as the sole bromine-containing compound. However, the formulation also may be prepared by optionally adding other bromine-containing compounds into the water during its preparation including, for example, hypobromous acid, bromide salts such as sodium bromide, bromites, bromates, and bromous acid, although these exemplified compounds are relatively unstable in aqueous formulations. When included in addition to the hypobromite salt, the other optional bromine-containing compounds are present in an amount less than the amount (by weight) of the hypobromite salt, and are desirably limited to from about 0.01, about 0.1, about 1, about 5, about 10, or about 50 ppm to no more than about 300, about 200, about 100, about 50, about 10, about 5, about 1 ppm or about 0.1 ppm, based on the weight of the formulation.

Each of the inventive aqueous formulations also is prepared with a chlorite salt. This salt may include one of a variety of cations, e.g., alkali and alkali earth metals salts, and is desirably a sodium salt. In preparing the formulations, the chlorite salt and may be present in the formulation in weight amounts ranging from about 1, about 100, about 500, about 1,000, about 2,000, about 4,000, about 5,000, about 7,000, about 10,000, about 15,000 or about 20,000 ppm to about 30,000, about 40,000, about 50,000, about 60,000, about 70,000, about 80,000, about 90,000, about 100,000, about 120,000 or about 150,000 ppm, based on the total weight of the formulation, and desirably ranges from about 25, 50, 100, 150, 200, 250, 300 or 400 to about 500, 600, 700, 800, 900 or 1,000 ppm, based, again, on the total weight of the formulation. In the inventive formulations, and as part of the numerous reactions that occur therein, at least a portion of the chlorite salt will be subject to a reaction that provides chlorine dioxide, as well as, if sodium chlorite is used, sodium chloride. The sodium chloride is believed to catalyze the formation of a hypochlorite, which in turn enhances the production of chlorine dioxide, the latter which is desirably present in the formulation when used.

The formulations are preferably prepared using sodium chlorite as the sole chlorite, and more preferably as the sole chlorine-containing compound. However, the formulation also may be prepared by adding other optional chlorine-containing compounds into the water during its preparation including, for example, sodium hypochlorite, but desirably excludes chlorine *per se* and hypochlorites. The inclusion of sodium hypochlorite in particular should be limited as described herein, as it adversely affects formulation taste and odor. When included in addition to the chlorite salt, the other optional chlorine-containing compounds are present in the formulation in an amount that is significantly less than the amount (by weight) of the chlorite salt, and are desirably limited to from about 0.01, about 0.1, about 1, about 5, about 10, or about 50 ppm to no more than about 300, about 200, about 100, about 50, about 10, about 5, about 1 ppm, or about 0.1 ppm.

As discussed further herein, the pH of the formulation should be carefully controlled. There are a variety of reasons for controlling the pH. For example, the pH of the formulations will affect the conversion of the chlorite salt, and, if included, sodium hypochlorite, to chlorine dioxide. The pH also will affect the stability of the hypobromite salt, wherein a relatively higher pH within the ranges described herein is more desirable, e.g., at least about 5, and more desirably at least about 6. The pH of the formulation ranges from about 4 to about 9, more desirably from about 5 to about 8, even more desirably from about 6 to about 8, and preferably from about 6 to about 7. In certain embodiments, the formulations have pH that is non-acidic, and may range from about 7 to about 7.5, 8, 8.5, 9 or 9.5 or 10, even more desirably from about 7.5 to about 8, 8.5 or 9, preferably from about 7.5 or 8 to about 8.5, and more preferably about 8.

The pH of the formulations is provided via the inclusion of a pH adjusting agent. This agent, added to the water during preparation of the formulation, may be any suitable non-toxic ingredient, or combination of ingredients, capable of providing the desired pH in the formulation when included in a particular amount. This being said, the direct addition of acids as a means of controlling pH is not preferred, because this can adversely affect the formation of chlorine dioxide. Thus, and preferably, the pH adjusting agent is a buffer, such as, e.g., sodium citrate and citric acid, sodium carbonate and citric acid, and will be included in an amount sufficient to provide the formulation with the desired pH. As the pH in the canine oral cavity will change with different stages of periodontal disease, the formulation, to be most effective, should provide antimicrobial, antiviral, and antifungal activity on the canine teeth and surrounding soft tissues over a range of pH values, and desirably at non-acidic pH levels.

The formulation described herein is also stable, retaining its effectiveness in reducing the quantity of bacteria, viruses, and/or fungi on canine teeth and/or in the surrounding soft tissues for at least about 7 days, more desirably for at least about 14 days, and even more desirably for at least about 30, 45, 60, 90, 120, 150 or 180 days.

In a preferred embodiment, the formulation is an aqueous solution or dispersion comprising, consisting essentially of, or consisting of sodium hypobromite in a weight amount ranging from about 1,000, about 1,250, about 1,500, about 1,750 or about 2,000 ppm to about 3,000, about 5,000, about 10,000, about 15,000, about 20,000, about 25,000, about 30,000, about 40,000, about 50,000 or about 80,000 ppm; sodium chlorite in an amount ranging from about 5,000, about 7,000, about 10,000, about 15,000 or about 20,000 ppm to about 50,000, about 60,000, about 70,000, about 80,000, about 90,000 or about 100,000 ppm, all weight amounts being those used to prepare the formulation and are based on the total weight of the formulation; water; and a pH adjusting agent, wherein the resulting formulation has a pH of from about 6 to about 8, as well as one more optional components and/or ingredients as described herein, and where desirably the formulation excludes various other components as described herein.

In yet another embodiment, the formulation is an aqueous solution or dispersion comprising, consisting essentially of, or consisting of sodium hypobromite in a weight amount ranging from about 1, 10, 25, 50, 75, 100 or 125 to about 150, 250, 500, 750, 1,000, 1,200 or 1,500; sodium chlorite in an amount ranging about 25, 50, 100, 150, 200, 250, 300 or 400 to about 500, 600, 700, 800, 900 or 1,000 ppm, all weight amounts being those used to prepare the formulation and are based on the total weight of the formulation; water; and a pH adjusting agent, wherein the resulting formulation has a pH of from about 6 to about 8, as well as one more optional components and/or ingredients as described herein, and where desirably the formulation excludes various other components as described herein.

The amount of the hypobromite and chlorite ions in the formulations after their preparation may be determined via any suitable analytical protocol, e.g., spectrophotometric, ion chromatography, HPLC, ICP and wet chemistry methods. Illustrative of one such protocol for determination of the amount of hypobromite ion via wet chemistry (and, thus, sodium hypobromite) in a solution is as follows. In a first titration (Titration I), a known volume (4 ml) of a solution prepared from sodium hypobromite (and, if desired, sodium bromate) is provided. 25 ml of deionized water (DI) is added to the 4ml solution and mixed. The resulting 29 ml of solution is titrated with 0.02M Na₂S₂O₃ (thiosulfate) until the solution turns yellow. 5 ml of a starch solution is then added, and titration is continued in the blue-colored solution until the blue-coloration disappears. In a second titration (Titration II), 4 ml of the same solution prepared from sodium hypobromite as used in Titration I is mixed with 6 ml of DI water. 1 g of ammonium sulfate is added to this mixture, which is then allowed to sit for 15 minutes. Thereafter, 3 g of KI and 10 ml of 2M H₂SO₄ are added, and the resulting mixture is allowed to sit for 10 mins. Titration is then conducted according to Titration I. The following formula allows for the calculation of the amount of hypobromite ion in the solution: Weight % hypobromite = (0.59445)(10/volume)(Titrate I volume - Titrate II volume)(Thiosulfate)(Molarity).

The formulations may desirably exclude various components, e.g., there is desirably no more than 1 ppm, more desirably no more than 0.1 ppm, even more desirably no more than 0.01 ppm, preferably no more than 0.001 ppm, and more preferably no detectable amount, based on the weight of the formulation, of these components. Illustrative of one class of components that are desirably excluded include certain components that can be oxidized, because the formulations contain relatively strong chemical oxidizers. Examples of these components include, e.g., chlorhexidine gluconate, EDTA, quaternary ammonium compounds (e.g., cetylpyridinium chloride (CPC)), chlorhexidine gluconate (CHG), certain surfactants, emollients, alcohols and essential oils. Although some quaternary ammonium compounds do possess antiseptic properties, e.g., CPC, they are not essential for the effectiveness of the formulation described herein. In this regard, a mouth rinse containing CPC (0.07%, Crest^{®} Pro-Health) as the active ingredient was found to not provide any statistically significant benefit over a mouth rinse which includes essential oils (Listerine^{®}) as the active ingredient. *See* Albert-Kiszely et al., "Comparison of the Effects of Cetylpyridinium Chloride with an Essential Oil Mouth Rinse on Dental Plaque and Gingivitis - a Six-month Randomized Controlled Clinical Trial," J. Clin. Periodontol. 34(8): 658-67 (Aug, 2007). Hydrogen peroxide also should be excluded because it will cause the hypobromite salt to convert to (toxic) bromine.

A further ingredient desirably (and optionally) included certain embodiments of the invention, and desirably in formulations that will contact the oral mucosa, is hyaluronan. Hyaluronan is hygroscopic and viscoelastic, and is believed to provide an enhancement to the antimicrobial properties of the formulations, as well as an anti-inflammatory relative to oral mucosa. The latter effect is especially desirable when the formulations are applied onto oral mucosa afflicted with periodontal disease.

Hyaluronan is polymeric, and may be used in crosslinked or non-crosslinked form. Certain hyaluronans, i.e., relatively low molecular weight hyaluronans, may elicit an inflammatory response in tissue onto which it is applied. It is therefore believed to be desirable to utilize non-crosslinked hyaluronan having a (weight average) molecular weight greater than about 500,000, desirably from about 1 million (M) to about 6M, and more desirably from about 1.5 M to about 4M. When using crosslinked hyaluronan, relatively lower molecular weight polymers may be used, as the crosslinking compensates for the disadvantages associated with the low molecular weight polymers.

When included in the inventive formulations, hyaluronan may be added in the formulations in an amount ranging from about 500, 1000, 1500, 2000, 4000 or 6,000 to about 8,000, 9,000, 12,000 or 16000 ppm, based on the final formulation.

Other optional formulation components, one or more of which may be included in any embodiment of the invention described herein, include preservatives, flavorings, sweeteners, teeth brighteners/whiteners, fluoride salts (for tooth conditioning), saliva enhancers, cannabidiol (CBD) oil, and additional viscosity enhancers.

A preservative, as its name implies, is believed to assist in preserving the formulations over time by assisting in stabilizing the active ingredients, and possibly by interacting with the buffer. The preservative may be added to the formulation in any amount suitable to provide for the desired degree of preservation, and may be added in amounts ranging from about 1 ppm, about 100, about 500, about 1,000, about 2,500, about 5,000 to about 10,000, about 12,500, about 15,000, about 17,500, about 20,000, about 25,000 or about 30,000 ppm, based on the weight of the formulation. Suitable preservatives include BHT, BHA, sodium nitrate, sulfites and sodium benzoate and other food-safe preservatives, with sodium benzoate being preferred.

Flavorings, e.g., chicken, beef, bacon, liver or fish flavor, or mint or other herb extracts, may be added, as may sweeteners (known to be safe for canine consumption), e.g., honey, sugar, and certain artificial sweeteners, to enhance acceptance by a canine. While amounts used may vary, the amount of flavoring may range from about 0.001 wt.% to about 1 wt.%, and desirably from about 0.01 wt.% to about 0.5 wt.%, of the formulation, and the amount of sweetener may range from about 0.001 wt.% to about 0.1 wt.%, and desirably from about 0.01 wt.% and about 0.5 wt.%, based on the weight of the formulation. Teeth brighteners/whiteners, e.g., silica, as well as saliva enhancers, may be added. When included, teeth brighteners may range from about 0.001 wt.% to about 1 wt.% based on the weight of the formulation, saliva enhancers may range from about 0.001 wt.% to about 1 wt.% based on the weight of the formulation.

Viscosity enhancers (in addition to, or other than hyaluronan, that latter which inherently provides an increase in formulation viscosity) are included. The increased viscosity, provided by certain viscosity enhancers, was found to provide the formulation with advantages, including assisting in the ease of application of the formulations onto the canine mucosa, while also improving the residence time of the formulation on the mucosa when a mucoadhesive viscosity enhancer is used. Enhancers suitable for use in the formulations are well-known, e.g., celluloses, Carbopols, with Carbopols being preferred due to their mucoadhesive properties. The viscosity enhancers may be included in amounts sufficient to provide the formulations with a viscosity that may range from about 1 to about 50 centipoise (cP), desirably from about 1 to about 20, and most desirably from about 1 to about 10 cP. While a relatively high viscosity formulation, e.g., about 20 to about 50 cP may be desirable depending on the method of application to permit ease of application onto the oral surfaces, e.g., directly from a dispenser, a relatively low viscosity formulation, e.g., from about 1 to about 20 cP is desirable if the formulation is applied using an absorbent applicator, such as a cotton or synthetic swab. The viscosity of the formulations may be determined using a Brookfield DV2T rotational viscometer at 25°C, 60 rpm, LV #3 or #4 spindle, and a 600 mL low form Griffin beaker (or equivalent container with a diameter of 8.25 cm).

The formulations may be prepared in any suitable manner. In one embodiment, the formulations may be prepared by providing: an aqueous solution of the pH adjusting agent (if a buffer, at least the acidic component); an aqueous solution of the hypobromite salt (and, if desired, and a buffer is used, a conjugate base); and an aqueous solution of the chlorite salt. The water used is preferably deionized. The aqueous solution formed by the addition of the pH adjusting agent is added to the aqueous solution formed by the addition of the chlorite salt, with the resulting aqueous solution (having a pH desirably between about 5 and about 9, and more desirably between about 7 and about 8.5) added to the solution formed by the addition of the hypobromite salt. In another embodiment, the formulation may be prepared, and packaged, in two parts and mixed prior to use to provide for activation of the formulation. In this embodiment, the aqueous solutions formed by the addition of the hypobromite and the chlorite salts are combined into one composition, with the aqueous solution formed by the addition of the pH adjusting agent (if a buffer, at least the acidic portion thereof) being added thereto prior to use.

The formulation, when applied onto the oral mucosa, provides activity against the primary colonization of hydroxyapatite (tooth surfaces) by the following canine oral bacteria:
*Actinomyces canis*
*Bergeyella zoohelcum*
*Capnoctophaga*
*Corynebacterium*
*Moraxella*
*Neisseria animaloris*
*Neisseria shayeganii*
*Neisseria weaveri*
*Neisseria zoodegmatis*
*Pasteurella dogmatis*
*Stenotrophomonas*
as well as the following subgingival anaerobic gram-negative bacteria found in canine periodontitis:
   *Peptostreptococcus gingivalis*
   *Porphyromonas gingivalis*
   *Bacteriodes fragilis*
   *Prevotella intermedia*

In addition to the other advantages described herein, it is believed that the inventive formulation retards the development of, or treats, periodontitis and/or malodor by reducing the quantity of anaerobic bacteria (one or more as described herein) in the canine oral cavity, including those responsible for odor, for at least about 1, about 2, about 4, about 6, about 8, about 10 or about 12 hours after application. It is believed that the formulation oxidizes the sulfide gases in the oral cavity which reduces malodor, and also kills anaerobic bacteria present in the canine oral cavity, on contact.

The formulation may be applied directly into the oral cavity, onto the teeth and surrounding soft tissue (e.g., gums) and any other desired areas by any suitable means, e.g., via spray, soft applicator, smeared (if in the form of a thickened gel, dispersion or viscous solution), so as to cover the aforementioned surfaces in a gentle manner.

The formulation should be applied at least once per day, and may be applied twice per day, e.g., at about 6, about 8, about 10 or about 12 hours after the initial application. The quantity applied may vary depending on the size of the canine oral cavity, but generally should be sufficient to permit the formulation to completely contact all teeth and surrounding soft tissue associated with the potential development of, or existing, periodontal disease, e.g., from about 1 to about 20 ml, desirably from about 2 to about 15 ml. Because the formulation is effective on contact, the time needed for the formulation to reside on the teeth and/or surrounding soft tissue after application thereon is limited to the time required to reduce the bacterial, viral and/or fungal load to an acceptable level. Desirably, this period ranges from about 1 second to about one minute, and more desirably from about 5 seconds to about 30 seconds. Repeated application (2, 3 or 4 times) within a single treatment may be undertaken if desired to enhance efficacy.

The formula described herein, because of its oxidation potential, will provide a reduction, and preferably an elimination, of any pellicle resident on the teeth. As a consequence, the reformation of harmful concentrations of bacteria on the tooth surface will be retarded, thereby reducing the accumulation of pathogens responsible for periodontal disease and putrid (sulfide) odors.

In addition, and because the formulation is a relatively strong oxidizer, any additional ingredients used in the preparation of the formulation, generally, should be limited to those that do not adversely affect the oxidation potential of the formulation.

The formulations described herein, because of their ORP, will provide a reduction, and preferably an elimination, of bacteria within the oral cavity after application, and further delay its reformation. As a consequence, the buildup of bacteria on the tooth surface and surrounding tissue will be prevented or delayed, thereby reducing the rate of the subsequent accumulation of pathogens responsible for the conditions described herein.

In general, the ORP of the formulations should be at least about 650 mV, and may be at least about 650, 675, 700, 725, 750, 775, or 790 mV, but desirably should not exceed 800 mV. In one embodiment, and when the formulations are applied within the oral cavity, the ORP may range from about 700, 725 or 750 to about 800 mV, from about 725 or 750 to about 775 mV, or from about 700 or 725 to about 750 mV.

The ORP of a formulation may be assessed using any suitable device, e.g., Hach Model HQ80 using a platinum probe (sensor), while the formulation is under atmospheric pressure and at room temperature, e.g., between about 20°C and about 30°C. Because the response of the sensor can degrade over time, it is desirable to test the sensor using a standard solution to verify that it is giving the correct response, such as within +/-10 mV, prior to assessing the ORP of an inventive formulation.

The formulations contemplated by the invention may vary slightly depending on their intended use. By way of illustration only, the following table provides a description of ingredients and amounts thereof that may be used to prepare the formulations contemplated by the invention, and which may be used in the methods described herein.

The table set forth below describes ingredients and amounts thereof that provide formulations that may be useful in the various methods contemplated by the present invention.

| | Formulation 1 | | Formulation 2 | Formulation 3 | Formulation 4 |
|---|---|---|---|---|---|
| Ingredient³ | ppm | wt.% | ppm | ppm | ppm |
| DI water (1 L) | | | | | |
| Sodium chlorite | 500 | 0.05 | 150 - 1,500 | 300 - 1000 | 400 - 600 |
| Sodium hypobromite | 150 | 0.015 | 25 - 500 | 100 - 250 | 100 - 200 |
| Hyaluronan | 8,000 | 0.8 | 4000 - 16,000 | 6,000 - 12,000 | 7,000 - 9,000 |
| Carbopol 974P | 8,000 | 0.8 | 4000 - 16,000 | 6,000 - 12,000 | 7,000 - 9,000 |
| Sucralose | 300 | 0.03 | 50 - 1200 | 100 - 900 | 200 - 500 |
| Mint | 300 | 0.03 | 50 - 1200 | 100 - 900 | 200 - 500 |
| Sodium carbonate¹ | 1000 | 0.1 | 250 - 4000 | 500 - 2000 | 750 - 1500 |
| Citric acid¹ | 1000 | 0.1 | 250 - 4000 | 500 - 2000 | 750 - 1500 |
| Benzoic Acid² | 100 | 0.01 | 25 - 400 | 50 - 200 | 75 - 150 |
| | | | | | |
| pH | 7 - 8.5 | | 5 - 9 | 6 - 8.5 | 7 - 8.5 |

| | | | | | |
|---|---|---|---|---|---|
| Amounts may vary as needed to obtain desired pH. ²Added as a preservative. ³The amount of each ingredient identified in this example (other than water, and the buffer/preservative which are already variable) may be varied ±10 wt.%. | | | | | |

The methods of the present invention include a method for reducing plaque which comprises applying the inventive formulation onto at least one tooth in an amount and for a time sufficient to reduce the amount of plaque thereon. An assessment of the effectiveness of the formulation in reducing plaque may be conducted via any known means, including qualitatively via the use of an oral disclosing solution. These solutions, which include a dye that adheres to plaque, may be applied before and after application of the formulation onto the tooth to be treated, with the relative intensity of the dye on the tooth being compared before and after use. A relatively lower dye intensity after application of the formulation indicates a reduction in plaque. An assessment of plaque and overall periodontal health (and the effectiveness of the inventive formulations), before and after treatment with the inventive formulations, also may be measured via use of an assay for a bacterial species most often associated with periodontal disease in canines, Porphyromonas gulae.

The invention also contemplates a method for reducing the quantity of bacteria on at least one tooth, or in areas within the oral cavity, which comprises applying the inventive formulation onto the tooth or area of the oral cavity in an amount and for a time sufficient to reduce the quantity of bacteria thereon. Regarding the effectiveness of the formulation in reducing bacteria on the teeth, and in the oral cavity, any known method may be used, e.g., an oral disclosing dye because a reduction in plaque is also indicative of a reduction in bacteria, as well as using known DNA assays for undesired oral bacteria.

Further contemplated is a method for reducing malodor of the canine oral cavity which comprises applying the inventive formulation within the oral cavity in an amount and for a time sufficient to decrease malodor of the oral cavity. When using the formulation to reduce oral odors (malodors), a qualitative analysis includes assessment by normal human olfactory evaluation, by comparing the smell of the canine breath before and after application of the formulation.

The American Veterinary Dental College has developed a classification system for assessing periodontal disease (PD) in teeth using a scale of 0 to 4. Each tooth may be classified independently; it is possible, and indeed common, for different teeth to have different classifications. A classification of PD 0 is used when a canine tooth is clinically normal, wherein there is no gingival inflammation or periodontitis clinically present. In PD Stage 1, gingivitis only is present without attachment loss, and the height and architecture of the alveolar margin are normal. In PD Stage 2, which can be referred to as early periodontitis, there is less than 25% of attachment loss or, at most, there is a stage 1 furcation involvement in multirooted teeth and there are early radiologic signs of periodontitis, wherein the loss of periodontal attachment of less than 25% is measured either by probing of the clinical attachment level, or radiographic determination of the distance of the alveolar margin from the cemento-enamel junction relative to the length of the root. Stage 1 (F1) furcation exists when a periodontal probe extends less than half way under the crown in any direction of a multirooted tooth with attachment loss. In PD Stage 3, which can be referred to as moderate periodontitis, there exists 25-50% of attachment loss as measured either by probing of the clinical attachment level, radiographic determination of the distance of the alveolar margin from the cemento-enamel junction relative to the length of the root, or there is a stage 2 furcation involvement in multirooted teeth. Stage 2 furcation exists when a periodontal probe extends greater than half way under the crown of a multirooted tooth with attachment loss but not through and through. In PD Stage 4, which can be referred to as advanced periodontitis, there exists more than 50% of attachment loss as measured either by probing of the clinical attachment level, or radiographic determination of the distance of the alveolar margin from the cemento-enamel junction relative to the length of the root, or there is a stage 3 furcation involvement in multirooted teeth. Stage 3 furcation exists when a periodontal probe extends under the crown of a multirooted tooth, through and through from one side of the furcation out the other. *See* Wolf HF, Rateitschak EM, Rateitschak KH et al., Color Atlas of Dental Medicine: Periodontology (3rd ed. Stuttgart: Georg Thieme Verlag) 2005.

The formulations of the invention may be used in connection with a canine presenting any of the foregoing classifications of periodontal disease on at least one tooth, e.g., as a method of retarding the progression of PD from one stage to the next stage (relative to a canine wherein the formulation is not applied), or more commonly as sole or as co-therapy (with cleaning and/or extraction) for canines having at least one tooth classified in PD Stages 0, 1, 2, 3 or 4.

### EXAMPLE 1

An illustrative formulation suitable for use various methods of the present invention (e.g., for the treatment of periodontal disease) in ultrasonic cleaning (as a debridement solution) is provided by combining the ingredients as set forth in the following table.

| Ingredient | ppm | wt.% |
|---|---|---|
| DI water (1 L) | | |
| Sodium chlorite | 500 | 0.05 |
| Sodium hypobromite | 150 | 0.015 |
| Hyaluronan | 8000 | 0.8 |
| Carbopol 974P | 8000 | 0.8 |
| Mint | 300 | 0.030 |
| Sodium carbonate¹ | 1000 | 0.1 |
| Citric acid¹ | 1000 | 0.1 |
| Benzoic acid² | 100 | 0.01 |
| | | |
| pH | 6.5 - 8.5 | |

| | | |
|---|---|---|
| ¹Amounts may vary as needed to obtain desired pH. ²Added as a preservative. | | |

An exemplary process for preparing 1 L of this formulation is as follows. 400 ml DI water is added to a suitable (non-reactive) container. 8 g of Carbopol 974P is added to the water, with continuous stirring until homogenization occurs (about 24 hours). Flavoring (e.g., mint) and sweetener (e.g., sucralose) are then added with gentle mixing until dissolved (approximately 10 minutes). Sodium hypobromite is then added, and the pH adjusted if needed (preferably via a buffer) to between 6 and 8. Hyaluronan is added with stirring, this stirring continuing until the hyaluronan is homogenized. Sodium chlorite (anhydrous, ca. 80 - 98% purity) is then added, with stirring. The pH of this formulation may be assessed and may be adjusted via the buffer as needed to an optimal value of 6.5 - 8.5. A final ORP assessment should be undertaken to ensure the ORP of the mixture is at least 700 mV. Additional DI water is then added to bring the mixture to 1 L total volume, with the product then being protected from light (e.g., amber or opaque container) until used.

### EXAMPLE 2

An illustrative formulation suitable in ultrasonic cleaning (as a debridement solution) is provided by combining the ingredients as set forth in the following table.

| Ingredient | ppm | wt.% |
|---|---|---|
| DI water (1 L) | | |
| Sodium chlorite | 500 | 0.05 |
| Sodium hypobromite | 150 | 0.015 |
| Mint | 300 | 0.030 |
| Sodium carbonate¹ | 1000 | 0.1 |
| Citric acid¹ | 1000 | 0.1 |
| Benzoic acid² | 100 | 0.01 |
| | | |
| pH | 6.5 - 8.5 | |

| | | |
|---|---|---|
| ¹Amounts may vary as needed to obtain desired pH. ²Added as a preservative. | | |

Unless otherwise specified, all quantities described herein are by weight, and are based on the total weight of the formulation.

The use of the terms "a" and "an" and "the" and "at least one" and similar referents in the context of describing the invention (especially in the context of the following claims) are to be construed to cover both the singular and the plural, unless otherwise indicated herein or clearly contradicted by context. The use of the term "at least one" followed by a list of one or more items (for example; "at least one of A and B") is to be construed to mean one item selected from the listed items (A or B) or any combination of two or more of the listed items (A and B), unless otherwise indicated herein or clearly contradicted by context. The terms "comprising," "having," "including," and "containing" are to be construed as open-ended terms (i.e., meaning "including, but not limited to,") unless otherwise noted. Recitation of ranges of values herein are merely intended to serve as a shorthand method of referring individually to each separate value falling within the range, unless otherwise indicated herein, and each separate value is incorporated into the specification as if it were individually recited herein.

## Claims

1. A formulation prepared via mixing a plurality of components to provide an aqueous solution or dispersion, the components comprising a hypobromite salt, a chlorite salt, water, a pH adjusting agent, and a viscosity enhancer, wherein the resulting formulation has a pH of from about 4 to about 9.

2. The formulation of claim 1, wherein the hypobromite salt and the chlorite salt are sodium salts.

3. The formulation of claim 1 or claim 2, wherein the pH adjusting agent is a buffer.

4. The formulation of any of claims 1 to 3, wherein the amount of hypobromite salt used to prepare the formulation is about 1 to about 3,000 ppm, the amount of chlorite salt used to prepare the formulation is about 1 to about 20,000 ppm, based on the total weight of the formulation.

5. The formulation according to any preceding claim, wherein the formulation has a pH of from about 6 to about 8.5.

6. The formulation according to any preceding claim, further comprising one or more of a preservative, a flavoring, a sweetener, a teeth brightener or whitener, a fluoride salt, or a saliva enhancer.

7. The formulation of any preceding claim, wherein the formulation excludes one or more of cetylpyridinium chloride (CPC), chlorhexidine gluconate (CHG) and alcohols.

8. The formulation of any preceding claim, further comprising a cannabidiol oil.

9. The formulation of any preceding claim packaged within an opaque container.

10. A formulation of any of claims 1 to 9 for use in reducing the quantity of bacteria, virus and/or fungi on canine teeth and/or in the surrounding soft tissues for at least about 7 days.

11. The formulation for use of claim 10, wherein the bacteria is selected from one or more of:
Actinomyces canis
Bergeyella zoohelcum
Capnoctophaga
Corynebacterium
Moraxella
Neisseria animaloris
Neisseria shayeganii
Neisseria weaveri
Neisseria zoodegmatis
Pasteurella dogmatis
Stenotrophomonas
Peptostreptococcus gingivalis
Porphyromonas gingivalis
Bacteriodes fragilis, and
Prevotella intermedia.

12. A formulation of any of claims 1 to 9, for use in retarding the advance of, or treating, periodontitis and/or malodor in a canine.

13. The formulation for use of claim 12, wherein the periodontitis is Stage 1, 2, 3 or 4 periodontal disease.

14. A formulation of any of claims 1 to 9, for use in reducing the quantity of plaque on at least one tooth of a canine.

## Patentansprüche

1. Formulierung, hergestellt durch Mischen einer Vielzahl von Komponenten, um eine wässrige Lösung oder Dispersion bereitzustellen, wobei die Komponenten ein Hypobromitsalz, ein Chloritsalz, Wasser, ein pH-Einstellmittel und einen Viskositätsverstärker umfassen, wobei die resultierende Formulierung einen pH-Wert von etwa 4 bis etwa 9 aufweist.

2. Formulierung nach Anspruch 1, wobei das Hypobromitsalz und das Chloritsalz Natriumsalze sind.

3. Formulierung nach Anspruch 1 oder Anspruch 2, wobei das pH-Einstellmittel ein Puffer ist.

4. Formulierung nach einem der Ansprüche 1 bis 3, wobei die Menge an Hypobromitsalz, die zur Herstellung der Formulierung verwendet wird, etwa 1 bis etwa 3.000 ppm beträgt, die Menge an Chloritsalz, die zur Herstellung der Formulierung verwendet wird, etwa 1 bis etwa 20.000 ppm beträgt, bezogen auf das Gesamtgewicht der Formulierung.

5. Formulierung nach einem vorhergehenden Anspruch, wobei die Formulierung einen pH-Wert von etwa 6 bis etwa 8,5 aufweist.

6. Formulierung nach einem vorhergehenden Anspruch, ferner umfassend eines oder mehrere von einem Konservierungsmittel, einem Aroma, einem Süßungsmittel, einem Zahnaufheller oder -bleicher, einem Fluoridsalz oder einem Speichelverstärker.

7. Formulierung nach einem vorhergehenden Anspruch, wobei die Formulierung eines oder mehrere von Cetylpyridiniumchlorid (CPC), Chlorhexidingluconat (CHG) und Alkoholen nicht enthält.

8. Formulierung nach einem vorhergehenden Anspruch, ferner umfassend ein Cannabidiolöl.

9. Formulierung nach einem vorhergehenden Anspruch, verpackt in einem opaken Behälter.

10. Formulierung nach einem der Ansprüche 1 bis 9 zur Verwendung bei der Verminderung der Menge an Bakterien, Viren und/oder Pilzen auf Hundezähnen und/oder in den umgebenden Weichgeweben für mindestens etwa 7 Tage.

11. Formulierung zur Verwendung nach Anspruch 10, wobei die Bakterien ausgewählt sind aus einem oder mehreren von:
Actinomyces canis
Bergeyella zoohelcum
Capnoctophaga
Corynebacterium
Moraxella
Neisseria animaloris
Neisseria shayeganii
Neisseria weaveri
Neisseria zoodegmatis
Pasteurella dogmatis
Stenotrophomonas
Peptostreptococcus gingivalis
Porphyromonas gingivalis
Bacteriodes fragilis und
Prevotella intermedia.

12. Formulierung nach einem der Ansprüche 1 bis 9 zur Verwendung beim Verzögern des Fortschreitens oder zum Behandeln von Parodontitis und/oder Mundgeruch bei einem Hund.

13. Formulierung zur Verwendung nach Anspruch 12, wobei die Parodontitis eine Parodontose im Stadium 1, 2, 3 oder 4 ist.

14. Formulierung nach einem der Ansprüche 1 bis 9 zur Verwendung bei der Verminderung der Menge an Plaque auf mindestens einem Zahn eines Hundes.

## Revendications

1. Formulation préparée en mélangeant une pluralité de composants pour obtenir une solution ou une dispersion aqueuse, les composants comprenant un sel hypobromite, un sel chlorite, de l'eau, un agent d'ajustement du pH et un améliorateur de viscosité, dans laquelle la formulation résultante a un pH d'environ 4 à environ 9.

2. Formulation de la revendication 1, dans laquelle le sel hypobromite et le sel chlorite sont des sels de sodium.

3. Formulation de la revendication 1 ou la revendication 2, dans laquelle l'agent d'ajustement du pH est un tampon.

4. Formulation de l'une quelconque des revendications 1 à 3, dans laquelle la quantité de sel hypobromite utilisée pour préparer la formulation est d'environ 1 à environ 3 000 ppm, la quantité de sel chlorite utilisée pour préparer la formulation est d'environ 1 à environ 20 000 ppm, sur la base du poids total de la formulation.

5. Formulation selon l'une quelconque des revendications précédentes, ladite formulation ayant un pH d'environ 6 à environ 8,5.

6. Formulation selon l'une quelconque des revendications précédentes, comprenant en outre un ou plusieurs parmi un conservateur, un arôme, un édulcorant, un azurant ou un blanchisseur dentaire, un sel fluorure ou un activateur de salive.

7. Formulation de l'une quelconque des revendications précédentes, ladite formulation excluant un ou plusieurs parmi le chlorure de cétylpyridinium (CPC), le gluconate de chlorhexidine (CHG) et les alcools.

8. Formulation de l'une quelconque des revendications précédentes, comprenant en outre une huile de cannabidiol.

9. Formulation de l'une quelconque des revendications précédentes, conditionnée dans un récipient opaque.

10. Formulation de l'une quelconque des revendications 1 à 9, destinée à être utilisée pour réduire la quantité de bactéries, de virus et/ou de champignons sur les dents et/ou dans les tissus mous environnants de chien pendant au moins environ 7 jours.

11. Formulation destinée à être utilisée selon la revendication 10, dans laquelle la bactérie est choisie parmi une ou plusieurs parmi :
Actinomyces canis
Bergeyella zoohelcum
Capnoctophaga
Corynebacterium
Moraxella
Neisseria animaloris
Neisseria shayeganii
Neisseria weaveri
Neisseria zoodegmatis
Pasteurella dogmatis
Stenotrophomonas
Peptostreptococcus gingivalis
Porphyromonas gingivalis
Bacteriodes fragilis, et
Prevotella intermedia.

12. Formulation de l'une quelconque des revendications 1 à 9, destinée à être utilisée pour retarder l'évolution de la parodontite et/ou de la mauvaise haleine chez un chien ou pour les traiter.

13. Formulation destinée à être utilisée selon la revendication 12, ladite parodontite étant une maladie parodontale de stade 1, 2, 3 ou 4.

14. Formulation de l'une quelconque des revendications 1 à 9, destinée à être utilisée pour réduire la quantité de plaque sur au moins une dent d'un chien.
